# EUROPEAN PATENT APPLICATION

(11) **EP 2 361 918 A1**
(43) Date of publication of application: **31.08.2011**
(21) Application number: 09827450.9
(22) Date of filing: 16.10.2009
(51) Int. Cl.: C07F 9/09, A61K 9/16, A61K 31/661, A61K 47/18, A61K 47/22, A61K 47/24, A61K 47/34, A61P 7/02, A61P 43/00

(54) **NOVEL PROSTAGLANDIN E1 DERIVATIVE, AND NANOPARTICLE HAVING SAME ENCAPSULATED THEREIN**

(30) Priority: 18.11.2008 JP 2008294167
(71) Applicant: LTT Bio-Pharma Co., Ltd., Minato-ku Tokyo 105-0022 (JP)
(72) Inventor: MIZUSHIMA, Toru, Kumamoto-shi Kumamoto 862-0975 (JP); OHTSUKA, Masami, Kumamoto-shi Kumamoto 860-0004 (JP); OKAMOTO, Yoshinari, Kamimashiki-gun Kumamoto 861-2222 (JP)
(74) Representative: Albrecht, Thomas
(86) International application number: PCT/JP2009/067885
(87) International publication number: WO 2010/058669

(57) **Abstract**

A PGE1 derivative is provided which has an excellent sustained, slow-release PGE1 action. In addition, a PGE1-derivative-containing nanoparticle produced using this PGE1 derivative is provided, which effectively targets an affected site, has excellent drug slow-release properties, and has reduced side effects. This PGE1-derivative-containing nanoparticle is a nanoparticle containing a prostaglandin E1 derivative represented by the following formula (1) 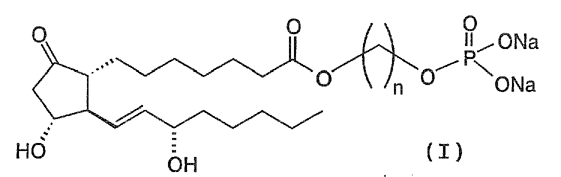 (wherein n denotes an integer of 1 to 12),
obtained by hydrophobicizing the prostaglandin E1 derivative with a metal ion, and reacting tne hydrophobicized prostaglandin E1 derivative with poly L-lactic acid or a poly(L-lactic acid/glycolic acid) copolymer and a poly DL- or L-lactic acid-polyethylene glycol block copolymer or a poly(DL- or L-lactic acid/glycolic acid)-polyethylene glycol block copolymer.

## Description

### TECHNICAL FIELD

The present invention relates to a novel prostaglandin E1 (PGE1) derivative and a nanoparticle having the same encapsulated therein.

### BACKGROUND ART

Prostaglandins (PG) are a group of physiological substances that have a prostanoic acid skeleton. Prostaglandins are one kind of eicosanoid that are biosynthesized from arachidonic acid, and are compounds having various strong physiological activities.
Various prostaglandin derivatives have hitherto been discovered. Among these, prostaglandin E1 (PGE1) is known to have a strong vascular dilatation action and platelet aggregation action, and based on these, a therapeutic action on chronic arterial occlusive disease. Several PGE1 derivatives are already being clinically used.

The present applicant has in the past synthesized several PGE1 derivatives and investigated their pharmacological action. One example of these is a PGE1 derivative represented by the development code number AS013, which is a PGE1 prodrug obtained by esterification of PGE1 so that it has an increased lipid solubility (Patent Document 1). This AS013 is a compound designed to be efficiently converted into PGE1 in the body. Since AS013 is a PGE1 prodrug, AS013 is known to have the vascular dilatation action, platelet aggregation action, therapeutic action on chronic arterial occlusive disease and the like that PGE1 possesses. Further, AS013 is also known to have an angiogenic action.
Another example of a PGE1 derivative synthesized by the present applicant is a lipoid preparation that embeds AS013 in fat particles in order to increase local transitivity. Since this lipoid preparation has excellent local affinity and clustering properties of AS013, and is gradually converted into PGE1, the lipoid preparation has an excellent sustained action (Patent Document 2).

In their synthesis and search of these PGE1 derivatives, the present inventors continued their investigation of the synthesis of a compound having a sustained, slow-release PGE1 action as a PGE1 derivative. As a result, the present inventors discovered that a very effective prodrug can be obtained by producing PGE1 as a phosphate derivative.
Further, the present inventors also confirmed that by producing such a PGE1 derivative as a nanoparticle enables excellent cellular uptake and an effective PGE1 action to be exhibited, thereby completing the present invention.
Patent Document 1: Japanese Patent No. 2849608
Patent Document 2: International Publication WO 1999/9992

### DISCLOSURE OF INVENTION

### PROBLEMS TO BE SOLVED BY THE INVENTION

Accordingly, it is an object of the present invention to provide a PGE1 derivative which has an excellent sustained, slow-release PGE1 action. In addition, it is also an object of the present invention to provide a PGE1-derivative-containing nanoparticle produced using this PGE1 derivative, which effectively targets an affected site, has excellent drug slow-release properties, and has reduced side effects.

### MEANS FOR SOLVING THE PROBLEMS

To resolve the above-described problems, a first basic aspect of the present invention is a prostaglandin E1 derivative represented by the following formula (I):

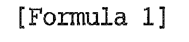

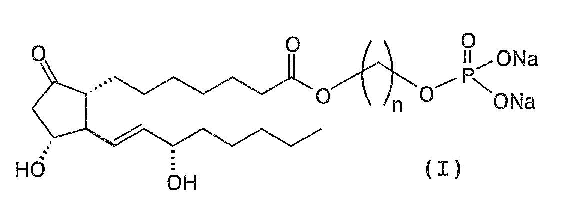

(wherein n denotes an integer of 1 to 12).

More preferably, the present invention is a prostaglandin E1 derivative represented by the above formula (I), wherein n is 2.

Further, as a second basic aspect, the present invention provides a nanoparticle containing a prostaglandin E1 derivative represented by the above formula (I). More specifically, the present invention is a nanoparticle containing a prostaglandin E1 derivative obtained by hydrophobicizing the prostaglandin E1 derivative represented by the formula (I) with a metal ion, and reacting the hydrophobicized prostaglandin E1 derivative with poly L-lactic acid or a poly(L-lactic acid/glycolic acid) copolymer and a poly DL- or L-lactic acid-polyethylene glycol block copolymer or a poly(DL- or L-lactic acid/glycolic acid)-polyethylene glycol block copolymer.

Preferably, the present invention is the above-described nanoparticle containing a prostaglandin E1 derivative, produced by further mixing with a basic low-molecular-weight compound, and then further adding a surfactant. Furthermore, in the nanoparticle containing a prostaglandin E1 derivative, the metal ion is one kind or two or more kinds of a zinc ion, an iron ion, a copper ion, a nickel ion, a beryllium ion, a manganese ion, and a cobalt ion.

More preferably, the present invention is a nanoparticle containing a prostaglandin E1 derivative, wherein the poly DL- or L-lactic acid-polyethylene glycol block copolymer or the poly(DL- or L-lactic acid/glycolic acid)-polyethylene glycol block copolymer has a weight average molecular weight of 3,000 to 30,000.

Further, the present invention is a nanoparticle containing a prostaglandin E1 derivative, wherein the basic low-molecular-weight compound is one kind or two or more kinds selected from (dimethylamino)pyridine, pyridine, piperidine, pyrimidine, pyrazine, pyridazine, quinoline, quinuclidine, isoquinoline, bis(dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylamine, dimethylamine, trimethylamine, triethylenediamine, diethylenetriamine, ethylenediamine, and trimethylenediamine.

In addition, the present invention is a nanoparticle containing a prostaglandin E1 derivative, wherein the surfactant is one kind or two or more kinds selected from phosphatidylcholine, polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (80) octylphenyl ether, polyoxyethylene (20) cholesterol ester, lipid-polyethylene glycol, polyoxyethylene hydrogenated castor oil, and a fatty acid-polyethylene glycol copolymer.

Most preferably, the present invention is a nanoparticle containing a prostaglandin E1 derivative having a particle diameter of 20 to 300 nm.

### EFFECT OF THE INVENTION

According to the present invention, a PGE1 derivative is provided as a prodrug that has an excellent sustained PGE1 action and that is effectively converted into PGE1.
The PGE1 derivative provided by the present invention can be synthesized from PGE1 by a simple process, yet is easily converted into PGE1. Further, the conversion of the PGE1 derivative is sustained, and the PGE1 derivative has excellent storage stability. Consequently, a particular feature of the PGE1 derivative is that it exhibits a slow-release PGE1 action.

Further, since the PGE1 derivative provided by the present invention can be easily converted into a hydrophobic metal salt, the PGE1 derivative can be used to produce a nanoparticle by using poly L-lactic acid or a poly(L-lactic acid/glycolic acid) copolymer and a poly DL- or L-lactic acid-polyethylene glycol block copolymer or a poly(DL- or L-lactic acid/glycolic acid)-polyethylene glycol block copolymer.
Consequently, the obtained nanoparticle is very stable, and the life of the PGE1 derivative in the particle is sustained over a prolonged period. Therefore, the nanoparticle provided by the present invention has excellent drug slow-release properties, and as a result, is very effective in terms of ability to achieve a sustained PGE1 action.

### BRIEF DESCRIPTION OF THE DRAWINGS

Fig. 1 is a graph illustrating the results of Example 2, which shows the conversion rate into PGE1 and an intermediate for pig liver esterase (PLE) treatment.
Fig. 2 is a graph illustrating the results of Example 2, which shows the conversion rate into PGE1 and an intermediate for human placental alkaline phosphatase (ALP) treatment.
Fig. 3 is a graph illustrating the results of Example 2, which shows the conversion rate into PGE1 and an intermediate for human serum treatment.
Fig. 4 is a graph illustrating the results of Example 2, which shows the conversion rate of a PGE1 (n = 2) phosphate derivative [PGE1(C2)PONa] into PGE1 for simultaneous PLE and ALP treatment.
Fig. 5 is a graph illustrating the results of Example 4, which shows changes in blood flow amount in a rat.
Fig. 6 is a graph illustrating the results of Example 4, which shows the conversion rate into PGE1 over time of a PGE1 derivative with rat plasma and human serum.
Fig. 7 is a graph illustrating the results of Example 7, which shows the particle diameter when PLA (molecular weight 5,000: PLA05) PLLA (molecular weight 5,000: PLLA05), and PLLA (molecular weight 20,000: PLLA20) are used as a base.
Fig. 8 is a graph illustrating the results of Example 7, which shows the PGE1 derivative encapsulation rate.
Fig. 9 is a graph illustrating the results of Example 7, which shows the results of an in vivo release test.
Fig. 10 is a graph illustrating the results of Example 8, which shows the pH during particle production.
Fig. 11 is a graph illustrating the results of Example 8, which shows the particle diameter of the produced particles.
Fig. 12 is a graph illustrating the results of Example 8, which shows the PGE1 derivative encapsulation rate of the produced particles.
Fig. 13 is a graph illustrating the results of Example 9, which shows the particle diameter.
Fig. 14 is a graph illustrating the results of Example 9, which shows the encapsulation rate.
Fig. 15 is a graph illustrating the results of Example 9, which shows the results of an in vitro release test.
Fig. 16 is a graph illustrating the results of Example 10, which shows the results of stability under 37°C conditions.
Fig. 17 is a graph illustrating the results of Example 10, which shows the results of stability under 4°C conditions.

### DESCRIPTION OF REFERENCE NUMERALS

In the drawings, C2, C3, C4, C6, C8, and C12 represent PGE1(C2)PONa, PGE1(C3)PONa, PGE1(C4)PONa, PGE1(C6)PONa, PGE1(C8)PONa, and PGE1(C12)PONa, respectively.

### BEST MODE FOR CARRYING OUT THE INVENTION

A basic mode of the present invention is a phosphate derivative of the PGE1 represented by the above formula (I).
Although various PGE1 derivatives have previously been proposed, the present invention is the first to provide PGE1 as a phosphate derivative. Therefore, such a PGE1 derivative is also a novel compound.

The PGE1 derivative provided by the present invention, namely, a PGE1 phosphate derivative, can specifically be prepared by the following production method.
The following chemical equation illustrates this preparation method in terms of a chemical reaction equation.

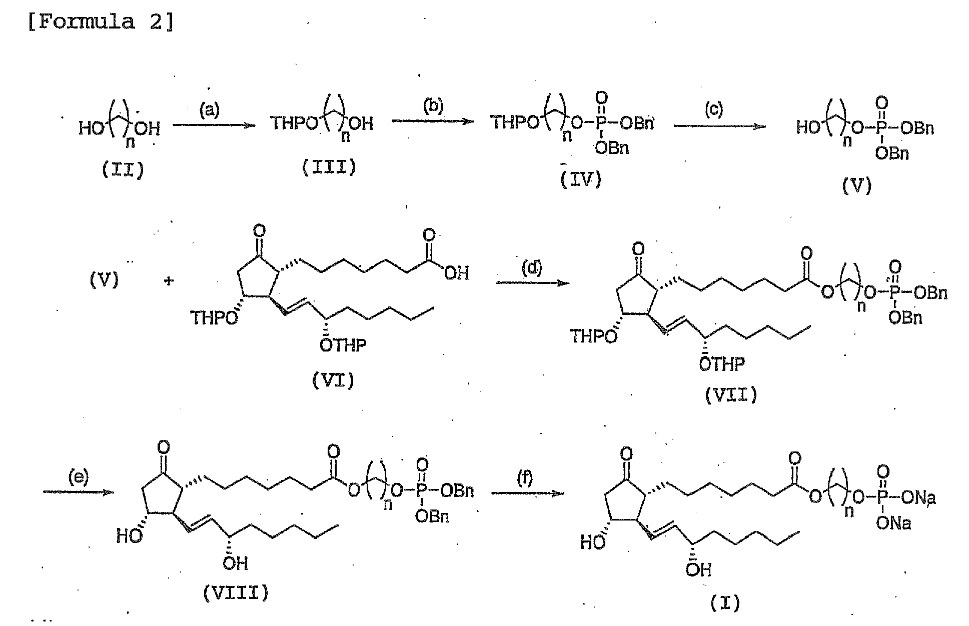

In the above chemical formula, n denotes an integer of 1 to 12, THP represents a tetrahydropyranyl group, and Bn represents a benzyl group. Further, (a) to (f) represent the respective steps.

Specifically, a compound (III) that is protected by a tetrahydropyranyl group (THP) is obtained by reacting one of the hydroxyl groups (OH groups) of the corresponding alkanediol compound (II) using 3,-4-dihydro-2H-pyran and aluminum chloride (Step a). The reaction employs the typical reaction conditions that are employed in introducing a tetrahydropyranyl group (THP group). Next, dibenzyl diisopropylphosphoramidite [((BnO)2-P(O)-N(iso-Pr)₂)₂] is reacted with the compound (III) in the presence of 1H-tetrazole to produce phosphorous acid on the other hydroxyl group of the compound (III). Then, the phosphorous acid is oxidized using a perbenzoic acid, such as m-chloroperbenzoic acid, to obtain a phosphoric acid derivative (IV) (Step b). Next, the tetrahydropyranyl group in the obtained phosphoric acid derivative (IV) is deprotected in a pyridinium p-toluenesulfonate ethanol solution to obtain a compound (V) (Step c).

Then, using the obtained compound (V), PGE1 is esterified to derive the intended PGE1 phosphate derivative according to the present invention. This process was carried out in the following manner.
Specifically, the compound (V) and a PGE1 compound (VI) are fused using, for example, EDC [1-ethyl-3-(3-dimethylaminopropyl)-carbodiimide] hydrochloride as a condensation agent, and 4-dimethylaminopyridine as a base, to derive a compound (VII) (Step d).
Next, the tetrahydropyranyl group in the obtained compound (VII) is deprotected with a suitable solvent, for example, acetic acid in a mixed solution of tetrahydrofuran-water, to obtain a compound (VIII) (Step e). Lastly, the benzyl group in the compound (VIII) is deprotected by reduction, and the resultant product is then converted into a sodium phosphate using sodium acetate, to derive the intended PGE1 derivative (I) according to the present invention. (Step f).
The debenzylation from the compound (VIII) into the PGE1 derivative represented by the formula (I), which is the intended compound of the present invention, can be carried out by, for example, catalytic reduction using 10% palladium-carbon in 1,4-cyclohexadiene/acetic acid.

The reaction conditions in the respective steps (a) to (f) in the preparation method of the above-described PGE1 derivative represented by the formula (I) according to the present invention can be carried out by variously applying the conditions which are described in common chemistry textbooks.

Examples of the thus-prepared PGE1 derivative according to the present invention may include the following.

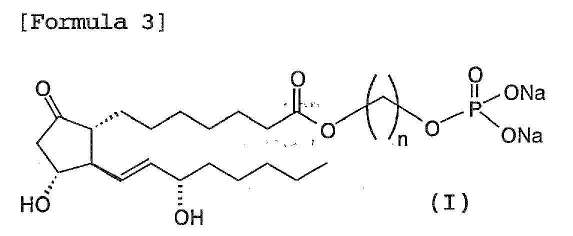

**[Table 1]**

| Compound (I) | Molecular weight | Properly | NMR: δ in CD₃OD |
|---|---|---|---|
| PGE1(C2)PONa n=2 | 522 | Yellowish oil | 0.83(3H,t), 1.15-1.52(18H,m), 1.96-2.29(5H,m), 4.14(2H,t) |
| PGE1(C3)PONa n=3 | 536 | Yellowish Oil | 0.81(3H,t), 1.22-1.44(18H,m), 1.84-2.26(7H,m), 4.09(2H,t) |
| PGE1(C4)PONa n=4 | 550 | Yellowish oil | 0.81(3H,t), 1.14-1.68(22H,m), 1.92-2.24(5H,m), 3.78(2H,q) |
| PGE1(C6)PONa n=6 | 578 | Yellowish Oil | 0.81(3H,t), 1.15-1.59(26H,m), 1.94-2.31(5H,m), 3.76(2H,q) |
| PGE1(C8)PONa n=8 | 606 | Yellowish oil | 0.81(3H,t), 1.15-1.52(30H,m), 1.95-2.28(5H,m), 3.76(2H,q) |
| PGE1(C12)PONa n=12 | 662 | Yellowish oil | 0.81(3H,t), 1.14-1.52(38H,m), 1.84-2.35(5H,m), 3.75(2H,q) |

The thus-prepared PGE1 derivative according to the present invention was easily converted into PGE1 itself in the body, and effectively exhibited the vascular dilatation action, platelet aggregation action and the like that PGE1 possesses (refer to the below-described working examples).

The present invention is also directed to a nanoparticle which encapsulates the PGE1 derivative prepared in the above-described manner. This nanoparticle is, specifically, prepared in the following manner.
Specifically, various research has previously been carried out regarding encapsulating a drug in a microparticle or a nanoparticle of a lactic acid/glycolic acid copolymer (hereinafter, sometimes referred to as "PLGA") or a lactic acid polymer (hereinafter, sometimes referred to as "PLA").
It is preferred to use these biodegradable polymers also in the production of the nanoparticle according to the present invention. Of these, it is especially preferred that the poly DL- or L-lactic acid-polyethylene glycol block copolymer (the DL-form may be referred to as "PDLLA-PEG" and the L-form may be referred to as "PLLA-PEG") or the poly(DL- or L-lactic acid/glycolic acid)-polyethylene glycol block copolymer (the DL-form may be referred to as "PDLLGA-PEG" and the L-form may be referred to as "PLLGA-PEG") is produced as a nanoparticle by using a copolymer obtained by reacting poly DL-lactic acid (which may be referred to as "PDLLA") or poly L-lactic acid (which may be referred to as "PLLA"), or a poly (DL-lactic acid/glycolic acid) copolymer (which may be referred to as "PDLLGA") or poly(L-lactic acid/glycolic acid) copolymer (which may be referred to as "PLLA") (these polymers will be referred to as "block A") with polyethylene glycol (which may be referred to also as "PEG") ("block B") in the presence of a condensation agent, such as ethylene dimethylaminopropyl carbodiimide.
These copolymers may be synthesized in accordance with the intended purpose, and similar commercially-available block copolymers may also be used.

The object of the present invention can be achieved with any of the following block copolymer configurations: A-B, A-B-A, and B-A-B. It is preferred that the weight average molecular weight of these block copolymers is 3,000 to 30,000.

The preparation of the nanoparticle provided by the present invention can be carried out by, specifically, hydrophobicizing the PGE1 derivative represented by the formula (I) with a metal ion, and reacting the hydrophobicized PGE1 derivative with poly L-lactic acid or a poly(L-lactic acid/glycolic acid) copolymer and a poly DL- or L-lactic acid-polyethylene glycol block copolymer or a poly(DL- or L-lactic acid/glycolic acid)-polyethylene glycol block copolymer.

More specifically, the PGE1 derivative represented by the formula (I) and the metal ion are mixed in a solvent, such as an organic solvent or an aqueous organic solvent, to produce a hydrophobicized drug. To this mixture, poly L-lactic acid or a poly(L-lactic acid/glycolic acid) copolymer and a poly DL- or L-lactic acid-polyethylene glycol block copolymer or a poly(DL- or L-lactic acid/glycolic acid)-polyethylene glycol block copolymer are added. The resultant solution is stirred, and then charged into water to diffuse, whereby the nanoparticle according to the present invention is prepared.

Further, the nanoparticle can also be prepared by simultaneously adding and mixing a solution in which the poly L-lactic acid or poly(L-lactic acid/glycolic acid) copolymer and the poly DL- or L-lactic acid-polyethylene glycol block copolymer or poly(DL- or L-lactic acid/glycolic acid)-polyethylene glycol block copolymer are dissolved, an aqueous solution of the PGE1 derivative represented by the formula (I), and a metal ion aqueous solution.

The used metal ion may be any of a zinc ion, an iron ion, a copper ion, a nickel-ion, a beryllium ion, a manganese ion, and a cobalt ion. One kind or two or more kinds of these ions are used in a water-soluble metal salt form. Of these ions, a zinc ion and an iron ion are preferred. These can be preferably used in the form of zinc chloride or iron chloride, for example.

The solvent used in the above-described reaction is an organic solvent, such as acetone, acetonitrile, ethanol, methanol, propanol, dimethylformamide, dimethylsulfoxide, dioxane, and tetrahydrofuran, or an aqueous solvent thereof. Of these solvents, acetone, dimethylformamide, dioxane, and tetrahydrofuran are preferred.

In the nanoparticle containing the PGE1 derivative represented by the formula (I) according to the present invention, the encapsulation rate of the PGE1 derivative in the nanoparticle can be increased by further mixing a basic low-molecular-weight compound. This encapsulation rate can be increased up to about 10%.
Examples of this basic low-molecular-weight compound include (dimethylamino)pyridine, pyridine, piperidine, pyrimidine, pyrazine, pyridazine, quinoline, quinuclidine, isoquinoline, bis(dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylanine, dimethylamine, trimethylamine, triethylenediamine, diethylenetriamine, ethylenediamine, and trimethylenediamine. Preferably, the basic low-molecular-weight compound is a secondary or a tertiary amine, and diethanol amine is especially preferable.

A surfactant may also be added to the thus-prepared nanoparticle containing the PGE1 derivative represented by the formula (I). Adding a surfactant stabilizes the produced nanoparticles and suppresses agglomeration among the nanoparticles. Therefore, adding a surfactant is preferred in terms of the preparation process of a nanoparticle-containing preparation.

Examples of the used surfactant include phosphatidylcholine, polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (80) octylphenyl ether, polyoxyethylene (20) cholesterol ester, lipid-polyethylene glycol, polyoxyethylene hydrogenated castor oil, and a fatty acid-polyethylene glycol copolymer. It is preferred to use one kind or two or more kinds of surfactant selected from among these examples.

The nanoparticles containing the PGE1 derivative provided by the present invention has a particle diameter in the range of 20 to 300 nm, and preferably from 50 to 200 nm. The particle diameter can be determined on the basis of the target site to which a particular drug is targeted.

The nanoparticle containing the PGE1 derivative represented by the formula (I) according to the present invention prepared in the above-described manner is separated from a solution or suspension containing the nanoparticles by a suitable purification technique, such as centrifugation, ultrafiltration, gel filtration, filter filtration, and fiber dialysis. The separated nanoparticles are then freeze-dried and stored.

It is preferred to add a stabilizing agent and/or a dispersing agent to the nanoparticles for freeze-drying, so that the freeze-dried preparation can be re-suspended prior to administration. Preferred examples of such stabilizing agents and dispersing agents include sucrose, trehalose, and carboxymethylcellulose sodium.

The nanoparticle containing the PGE1 derivative represented by the formula (I) provided by the present invention can be used in a pharmaceutical product as a drug formulation for parenteral administration, such as an intravenous formulation, a local injection formulation, a nasal formulation, an ophthalmic formulation, an inhalation formulation, and a spray formulation. The features and advantages of the inventive nanoparticle can be more effectively exploited when the nanoparticle is used in an intravenous formulation.

Examples of the bases and other added components used to prepare these drug formulations for parenteral administration include bases and other added components that are pharmaceutically acceptable and commonly used. Specific examples include physiological saline; sugars, such as monosaccharides, disaccharides, sugar alcohols, and polysaccharides; polymer additives, such as hydroxyethylcellulose, hydroxypropylcellulose, and methylcellulose; and ionic or nonionic surfactants. These bases and added components may be appropriately selected and used on the basis of the dosage form.

### EXAMPLES

The present invention will now be described in more detail using the following examples. However, the present invention is not limited to these examples.

### Example 1: Production of PGE1 Derivative

The various-PGE1 derivatives listed in the above Table 1 were synthesized in accordance with the above-described production method.
More specifically, a solution of 1.7 mmol of the compound represented by the formula (II) in 10 mL of dichloromethane was mixed with 2.5 mmol of 1H-tetrazole and 3.4 mmol of N,N-dibenzyl diisopropylphosphoramidite, and the resultant mixture was stirred overnight at room temperature. Then, 3.4 mmol of m-chloroperbenzoic acid was added to the mixture, and the mixture was stirred for 30 minutes at room temperature. 30 mL of chloroform was then added to the mixture. The organic layer was washed three times with 10 mL of saturated sodium bicarbonate aqueous solution and brine, respectively, and the solvent was removed by distillation. The obtained residue was subjected to silica gel column chromatography, and eluted with ethyl acetate : hexane = 1:1 ∼ ethyl acetate, to obtain the corresponding compound represented by the formula (IV) as a colorless oily substance.

A mixture of 1.35 mmol of the obtained compound represented by the formula (IV) and 0.3 mmol of pyridinium p-toluenesulfonate in 5 mL of ethanol was stirred for 3 hours at 55°C. The solvent was removed by distillation. The obtained residue was then subjected to silica gel column chromatography, and eluted with ethyl acetate : hexane = 3:2 ∼ ethyl acetate, to obtain the corresponding compound represented by the formula (V) as a colorless oily substance.

Next, a mixture of 0.25 mmol of the obtained compound represented by the formula (V), 0.4 mmol of EDC [1-ethyl-3-(3-dimethylaminopropyl)carbodiimide] hydrochloride, 0.20 mmol of 4-dimethylaminopyridine, and the PGE1 derivative represented by the formula (VI) in 3 mL of dichloromethane was stirred for 10 minutes at room temperature. 30 mL of chloroform was added to the mixture. The organic layer was washed three times with 10 mL of saturated sodium bicarbonate aqueous solution and brine, respectively, and the solvent was removed by distillation. The obtained residue was subjected to silica gel column chromatography, and eluted with ethyl acetate : hexane = 1:1, to obtain the corresponding compound represented by the formula (VII) as a colorless oily substance.

0.052 mmol of the obtained compound represented by the formula (VII) was dissolved in a mixture of 1.8 mL of acetic acid, 0.46 mL of tetrahydrofuran and 1.8 mL of water, and the resultant mixture was stirred for 4 hours at 35°C. 5 mL of saturated sodium bicarbonate aqueous solution was added to the reaction product at 0°C, and then the mixture was extracted three times with 50 mL of ethyl acetate. The separated organic layers were combined, washed three times with 10 mL of brine, and the solvent was removed by distillation. The obtained residue was subjected to silica gel column chromatography, and eluted with ethyl acetate : hexane = 1:1, to obtain the corresponding compound represented by the formula (VIII) as a colorless oily substance.

0.026 mmol of the compound represented by the formula (VIII) was mixed into 64 mg of 10% palladium-carbon, 2.8 mL of 1,4-cyclohexadiene, 0.2 mL of acetic acid, and 5 mL of ethanol, and the resultant mixture was stirred for 2 hours at room temperature under a hydrogen gas atmosphere. The 10% palladium-carbon was separated by filtration, and the resultant product was washed with ethanol. The organic layers were combined and concentrated to obtain the intended compound represented by the formula (I) as a yellow oily substance.
The nature and NMR data of the obtained compound are shown in the above Table 1.

### Example 2: Conversion of Various PGE1 derivatives into PGE1 by Enzyme and Serum Treatment

The conversion rates of the various PGE1 derivatives synthesized in Example 1 into PGE1 and intermediates when treated with pig liver esterase (PLE), human placental alkaline phosphatase (ALP), and human serum was measured using HPLC.
Further, the conversion rate of PGE1 (n = 2) sodium phosphate [PGE1(C2)PONa] into PGE1 when simultaneously treated with PLE and ALP was also investigated.
In addition, as a control, the same treatments were also carried out on AS013, which is an ester derivative of PGE1.
The conversion rate when the pre-reaction PGE1 derivative was completely converted into PGE1 was taken as 100%.
The specific methods were as follows.

### Methods:

### [Human Serum Treatment]

A PGE1 derivative was mixed with 1 µL of a 100 mM ethanol solution and 99 µL of human serum, and the resultant mixture was incubated at 37°C. Then, twice the amount of methanol was added, and deproteinization was carried out for 30 minutes under ice. Next, the mixture was centrifuged for 10 minutes at 4°C and 13,200 rpm, and 200 µL of supernatant was dried using a centrifugal concentrator. To the dried sample, added were 300 µL of 10 µg/mL 3-phenylpropionate as an internal standard, and then 1.7 mL of 50 mM EDTA (pH 3.6). After 20 minutes, the solution was loaded into a C18 reversed-phase cartridge column (SepPack C-18), washed with 6 mL of ultrapure water, and then eluted with 3 mL of acetonitrile. The eluent was mixed with an equivalent amount of a solution of 0.2 mg/mL 9-antryldiazomethane (ADAM) in acetone. The resultant mixture was incubated for 18 hours at 37°C under light-shielding. After incubation, the amount of PGE1 in the sample was measured using a Super-ODS column and a fluorescence detector.

### [Pig Liver Esterase (PLE) Treatment]

A PGE1 derivative was mixed with 1 µL of a 100 mM ethanol solution, 0.75 µL of a pig liver esterase (PLE) solution, and 98.25 µL of 0.1 M tris-HCl (pH 7.4), and the resultant mixture was incubated at 37°C. Then, an equivalent amount of methanol was added, and deproteinization was carried out for 30 minutes under ice. Next, the mixture was centrifuged for 10 minutes at 4°C and 13,200 rpm, and 150 µL of supernatant was dried using a centrifugal concentrator. To the dried sample, added were 300 µL of 10 µg/mL 3-phenylpropionate as an internal standard, and then 1.7 mL of 50 mM EDTA (pH 3.6). After 20 minutes, the solution was loaded into a C18 reversed-phase cartridge column (SepPack C-18), washed with 6 mL of ultrapure water, and then eluted with 3 mL of acetonitrile. The eluent was mixed with an equivalent amount of a solution of 0.2 mg/mL 9-anthryldiazomethane (ADAM) in acetone. The resultant mixture was incubated for 18 hours at 37°C under light-shielding. After incubation, the amount of PGE1 derivative in the sample was measured using a Super-ODS column and a fluorescence detector.

### [Human Placental Alkaline Phosphatase (ALP) Treatment]

A PGE1 derivative was mixed with 1 µL of a 100 mM ethanol solution, 1 µL of a human placental alkaline phosphatase (ALP) solution, and 98 µL of 0.1 M tris-HCl (pH 7.4), and the resultant mixture was incubated at 37°C. Then, an equivalent amount of methanol was added, and deproteinization was carried out for 30 minutes under ice. Next, the mixture was centrifuged for 10 minutes at 4°C and 13,200 rpm, and the supernatant was analyzed by HPLC.

The HPLC analysis conditions were as follows.

### [HPLC Conditions]

The HPLC was carried out using a Waters Alliance HPLS system, with Empower used as the software. Used for the pump, autosampler and the like was a 2795 Separation module. The detector was a 2996 Photodiode Array Detector. The column was a 4.6 x 100-mm (2-um) TSKgel Super-ODS column. Acetonitrile was used for the mobile phase A, and 5 mM ammonium acetate solution was used for the mobile phase B. The mobile phase A was applied at a gradient of 25% for 1 minute, followed by 25 to 60% for 7 minutes, and then 60 to 100% for 5 minutes. The sample was isolated by holding under conditions of 100% for 7 minutes.
The flow rate was 0.5 mL/min, the sample injection amount was 10 µL, and the detection wavelength was 195 nm.

### Results:

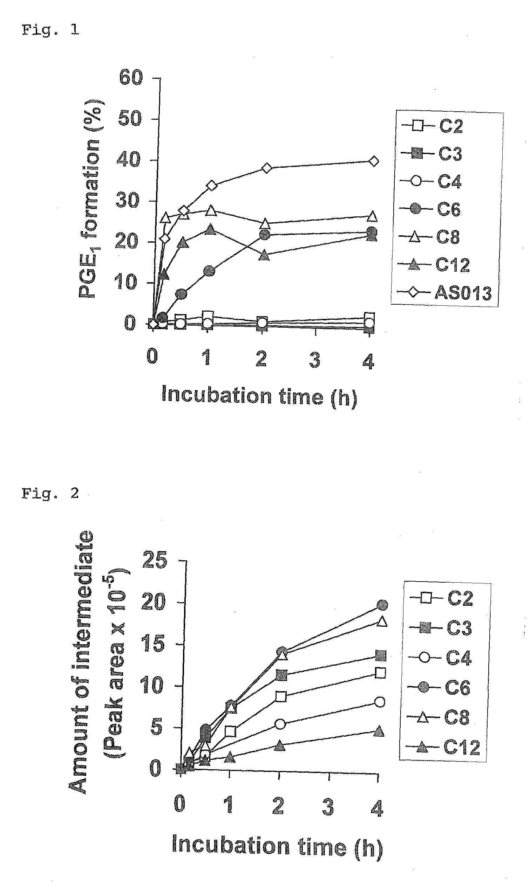

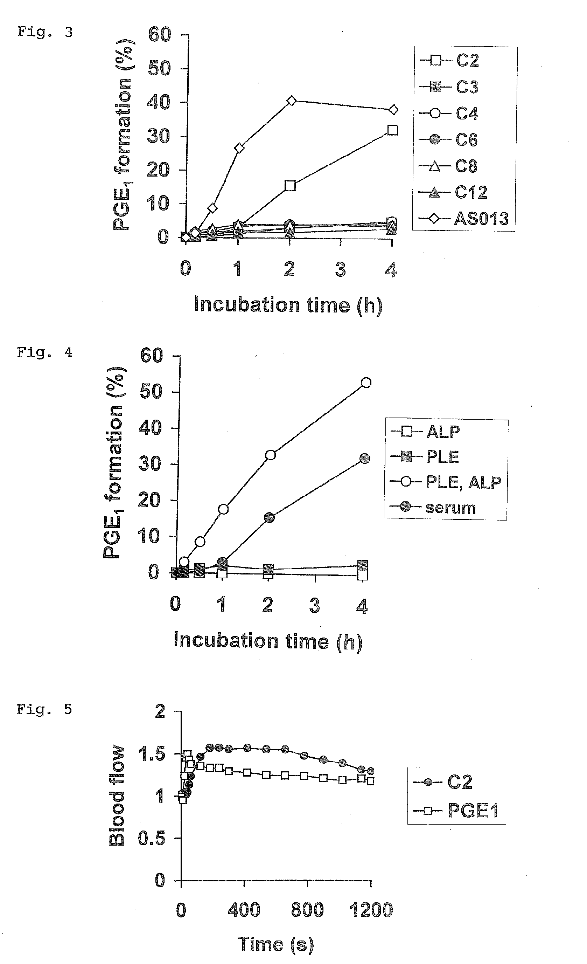

The results are illustrated in Figs. 1 to 4. Fig. 1 shows the conversion rate into PGE1 and an intermediate for the pig liver esterase (PLE) treatment. Fig. 2 shows the conversion rate into PGE1 and an intermediate for the human placental alkaline phosphatase (ALP) treatment. Fig. 3 shows the conversion rate into PGE1 and an intermediate for the human serum treatment.
Further, Fig. 4 shows the conversion rate of a PGE1 (n = 2) phosphate derivative [PGE1(C2)PONa] into PGE1 when simultaneously treated with PLE and ALP.
It can be seen that the PGE1 derivative provided by the present invention is converted into PGE1, which is the active substance, in a sustained manner.

### Example 3: Results of PGE1 Derivative Against Platelet Aggregation Induced by ADP (Adenosine Diphosphate)

Aggregation was induced by incubating a fraction (PRP) rich in platelets and a PGE1 derivative at 37°C, and then adding adenosine diphosphate (ADP) (final concentration: 2 µM). After 3 minutes, the level of aggregation was measured using a platelet aggregometer.
The induced aggregation when physiological saline was used as a sample was taken as 100%. Based on this, the concentration of the PGE1 derivative that inhibited aggregation by 50% was measured.
In addition, as a control, the same treatments were also carried out on AS013, which is an ester derivative of PGE1.

The specific method was as follows.

### [Platelet Treatment]

Blood was obtained from a healthy person that had not taken medication for a week or more using 3.8% sodium citrate (1/9 the amount of blood) as an anticoagulant. The blood was centrifuged at 1,000 rpm for 15 minutes to obtain the platelet-rich plasma (PRP). The remaining blood was centrifuged at 3,000 rpm for 10 minutes to obtain the platelet-poor plasma (PPP).
Aggregation was induced by incubating 215 µL of the PRP at 37°C for 1 minute, adding with 10 µL of the sample, and then adding 25 µL of 20 µM ADP. After 3 minutes, the level of aggregation was measured using a platelet aggregometer (NKK Hematracer 6, Niko Bioscience Inc., PAC-8S).
The PPP was used as a control.
The induced aggregation when physiological saline was used as a sample was taken as 100%. Based on this, the concentration of the PGE1 derivative that inhibited aggregation by 50% was calculated.
The sample was used in the experiment by diluting a 25 mM ethanol solution to various concentrations with physiological saline.
The results are shown in Table 2.

**[Table 2]**

| Test Compound | ED₅₀(µM) | | |
|---|---|---|---|
| | Incubation | | |
| | None | 15 min. after | 30 min. after |
| PGE1 | 0.15 | 0.09 | 0.09 |
| PGE1(C2)PONa | >10 | 1.51 | 0.49 |
| ASO13 | 2.81 | 0.83 | 0.73 |

It can be seen that PGE1(C2)PONa, which is a PGE1 derivative according to the present invention in which in the formula (1) n = 2, exhibits a slow-release platelet aggregation action.

### Example 4: PGE1 Derivative Action for Increasing Rat Blood Flow

The blood flow amount in the footpad skin of an anesthetized Wister male rate was measured by a laser Doppler method. A PGE1 derivative [(n = 2): PGE1(C2)PONa] was administered via the tail vein. Changes in the blood flow amount were observed with the sample administration time set at 0 minutes.
Further, the conversion rate of the PGE1 derivative [(n = 2): PGE1(C2)PONa] into PGE1 when treated with rat plasma was investigated.
The conversion rate when the pre-reaction PGE1 derivative was completely converted into PGE1 was taken as 100%.

### Method:

The specific method was as follows.

### [Measurement of Blood Flow Amount]

The blood flow amount in the footpad skin of an anesthetized Wister male rate was measured by a laser Doppler method. The PGE1 derivative was administered via the tail vein.
The sample dose was 10 nmol/kg.
Changes in the blood flow amount were observed with the sample administration time set at 0 minutes.

### Results:

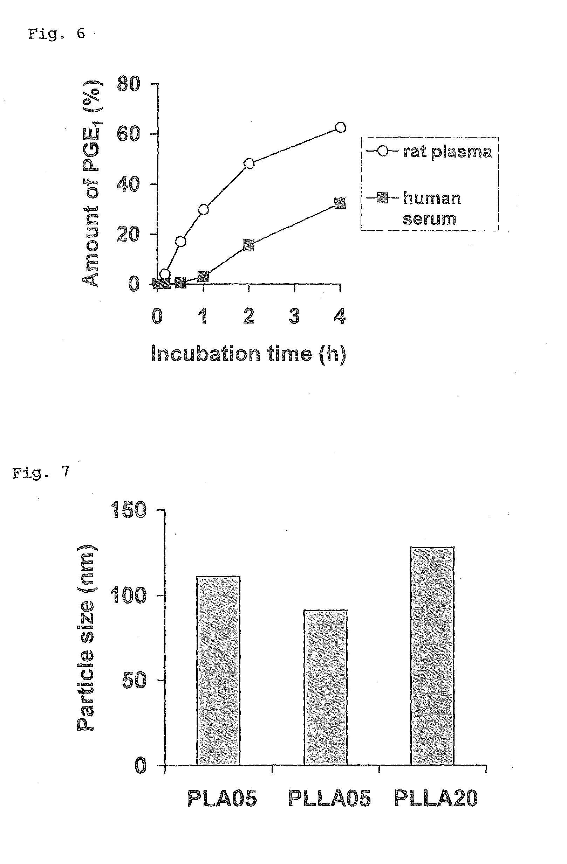

The results are shown in Figs. 5 and 6. Fig. 5 shows the change in blood flow amount in a rat. Based on these results, it is clear that the blood flow amount is maintained in a sustained manner with the PGE1 derivative represented by the formula (1) according to the present invention.
Fig. 6 shows the conversion rate of the PGE1 derivative into PGE1 over time with rat plasma and human serum. Based on these results, it can be seen that the conversion into PGE1 is carried out in a sustained manner.

Preparation of the nanoparticle according to the present invention, and the features of the prepared nanoparticle will now be described.

### Example 5: Synthesis of PLA-PEG Used in Nanoparticle Preparation

A polymerization tube was charged with 2 g of PEG, 2 to 6 g of dl-Lactide, and tin octylate (0.5 wt.%). The resultant mixture was thoroughly mixed, and then the air in the tube was evacuated with a hydraulic pump. The mixture was heated at 125°C with an oil bath to dissolve. The temperature was then increased to 160°C, and the mixture was allowed to react for 3 to 5 hours. The reaction product was cooled, and then dissolved in about 20 mL of dichloromethane. This mixture was then slowly charged into a large quantity of ice-cooled isopropanol to re-precipitate. The precipitate was suspended in water, then freeze-dried. The molecular weight of the obtained product was calculated on the basis of gel permeation chromatography (TSKgel α-4000, α-3000, α-2000) and NMR measurement.
The average molecular weight of the used PEG was about 5,000, and the average molecular weight of the PLA was about 3,000.

### Example 6: Nanoparticle Production Method

Particles were produced by an oil-in-water solvent diffusion method. PLLA was used by dissolving in 1,4-dioxane, PEG-PLA and DEA (diethanolamine) were used by dissolving in acetone, and zinc chloride and the PGE1 derivative were used by dissolving in ultrapure water. The total amount of the PLLA and PEG-PLA was 25 mg.
Mixed together were, in order, 22.5 µL of a PLLA solution, 27.5 µL of a PEG-PLA solution, 20.3 µL of 1 M zinc chloride aqueous solution, and 14.3µL of PGE1 derivative aqueous solution, and the resultant mixture was incubated for 10 minutes at room temperature. While stirring the mixture at 1,000 rpm, 25 mL of ultrapure water was added dropwise through a 26 G syringe at a rate of 48 mL/hour. After the dropping was finished, to the mixture added were 500 µL of 0.5 M sodium citrate aqueous solution (pH 7.4) and 12.5 µL of 200 mg/mL Tween 80 aqueous solution. As a result, excess zinc ions were chelated, thereby stabilizing particle diffusion. The particle suspension was concentrated by ultrafiltration using a Centriprep YM-50. The resultant product was further concentrated by adding 50 mM EDTA (pH 7) and ultrapure water, thereby purifying the particles.
The encapsulation amount of the PGE1 derivative in the particles was measured using a Super-ODS column and a fluorescence detector by reacting with ADAM.
The encapsulation rate of the PGE1 derivative in the particles was defined as the weight ratio of the PGE1 derivative with respect to the total particle weight.
The particle diameter and particle diameter distribution were measured by dynamic light diffusion.
These results are shown in the following Table 3.

**[Table 3]**

| Compound | Encapsulation Rate in Nanoparticle (wt%) | Particle size (nm) |
|---|---|---|
| PGE1(C2)PONa | 0.6 | 111 |
| PGE1(C3)PONa | 0.5 | 99 |
| PGE1(C4)PONa | 4.0 | 105 |
| PGE1(C6)PONa | 5.1 | 98 |
| PGE1(C8)PONa | 0.6 | 106 |
| PGE1(C12)PONa | 6.2 | 130 |

As can be clearly seen from the results in the table, the encapsulation rate of the PGE1 derivative in the particles was sufficient.

Further, the encapsulation amount of the PGE1 derivative in the nanoparticles was measured as follows.

### [Method for Measuring PGE1 Amount in Particles]

The PGE1 amount in the particles was measured as follows.
50 µL of a particle suspension was dried using a centrifugal concentrator. The dried particles were dissolved with 150 µL of 1,4-dioxane. To the resultant mixture added were 150 µL of 60 µg/mL 3-phenylpropionate as an internal standard, and then 1.7 mL of 50 mM EDTA (pH 3.6). After 20 minutes, the solution was loaded into a C18 reversed-phase cartridge column (SepPack C-18), washed with 6 mL of ultrapure water, and then eluted with 4.5 mL of acetonitrile. The eluent was mixed with an equivalent amount of a solution of 0.2 mg/mL 9-anthryldiazomethane (ADAM) in acetone. The resultant mixture was incubated for 18 hours at 37°C under light-shielding. After incubation, the amount of PGE1 was measured using a Super-ODS column and a fluorescence detector.
The encapsulation rate of the PGE1 in the particles was defined as the weight ratio of the PGE1 with respect to the total particle weight.

The PGE1-ADAM HPLC measurement was carried out as follows.
The HPLC was carried out using a Waters Alliance HPLS system, with Empower used as the software. Used for the pump, autosampler and the like was a 2795 Separation module. The detector was a 2996 Photodiode Array Detector and a 2475 Multi λ Fluorescence Detector. The column was a 4.6 x 100-mm (2 um) TSKgel Super-ODS column.
Acetonitrile was used for the mobile phase A, and ultrapure water was used for the mobile phase B.
The mobile phase A was applied at a gradient of 65% for 25 minutes, followed by 65 to 100% for 10 minutes. The sample was isolated by holding under conditions of 100% for 10 minutes.
The flow rate was 0.3 mL/min, the sample injection amount was 5 µL, the excitation-wavelength was 365 nm, and the detection wavelength was 412 nm.

### Example 7: Features of-Nanoparticles Having PGE1 Derivative [n = 2: PGE1(C2)PONa] Encapsulated Therein

Nanoparticles were produced by an oil-in-water solvent diffusion method.
The nanoparticles were produced using PLA (molecular weight: 5,000) or PLLA (molecular weight: 5,000 or 20,000) and PLA-PEG (molecular weight: 8,000) as a base.
The particle diameter and encapsulation rate of various nanoparticles were measured, and an in vivo release test was carried out.
The release test was carried out by incubating nanoparticles in a phosphate buffer solution at 37°C, and measuring the amount of PGE1 derivative [n =2: PGEI(C2)PONa] remaining in the particles at various times by HPLC.

These results are shown in Figs. 7 to 9.

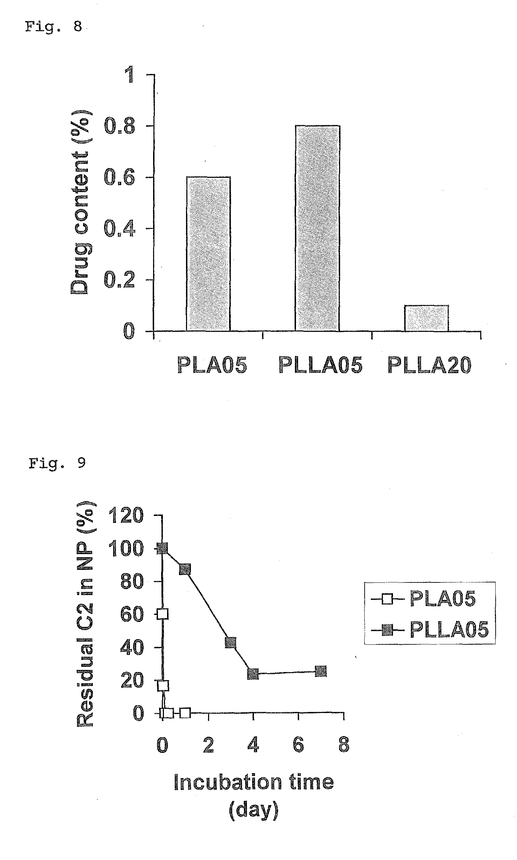

Fig. 7 shows the particle diameter, Fig. 8 shows the PGE1 derivative encapsulation rate, and Fig. 9 shows the results of the in vivo release test, when PLA (molecular weight 5,000: PLA05) PLLA (molecular weight 5,000: PLLA05), and PLLA (molecular weight 20,000: PLLA20) were used as a base.
Based on these results too, it is clear that when PLLA is used as the base, the obtained nanoparticles exhibit a good particle diameter, drug encapsulation rate, and slow-release properties.

### Example 8: Investigation of Conditions for Efficiently Encapsulating PGE1 Derivative [n =2: PGE1(C2)PONa] in PLLA Nanoparticles

Based on the results of Example 7, the conditions for even more efficient PGE1 derivative encapsulation were investigated.
More specifically, with the zinc amount fixed at 21.4 nmol during nanoparticle production, nanoparticles were produced while varying the amount of DEA (diethanolamine).

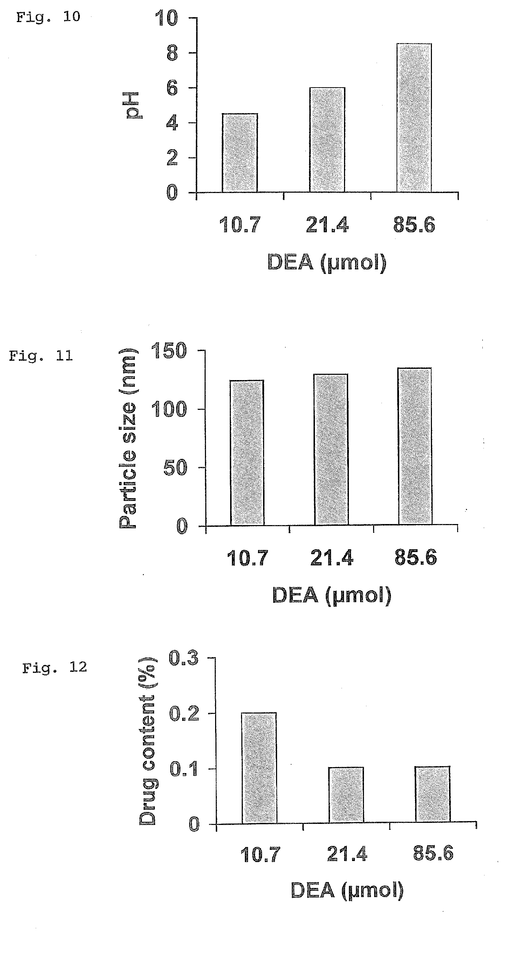

Fig. 10 shows the pH during particle production under various conditions. Fig. 11 shows the particle diameter of the produced particles. Fig. 12 shows the PGE1 derivative encapsulation rate of the produced particles.

### Example 9: Features of Nanoparticles Having PGE1 Derivative [n = 2: PGE1(C2)PONa] Encapsulated Therein After Changing Particle Production Conditions

Nanoparticles were produced using PLLA (molecular weight: 5,000 (PLLA05) or 20,000 (PLLA20)) and PLA-PEG (molecular weight: 8,000) as a base under conditions which varied the DEA amount.

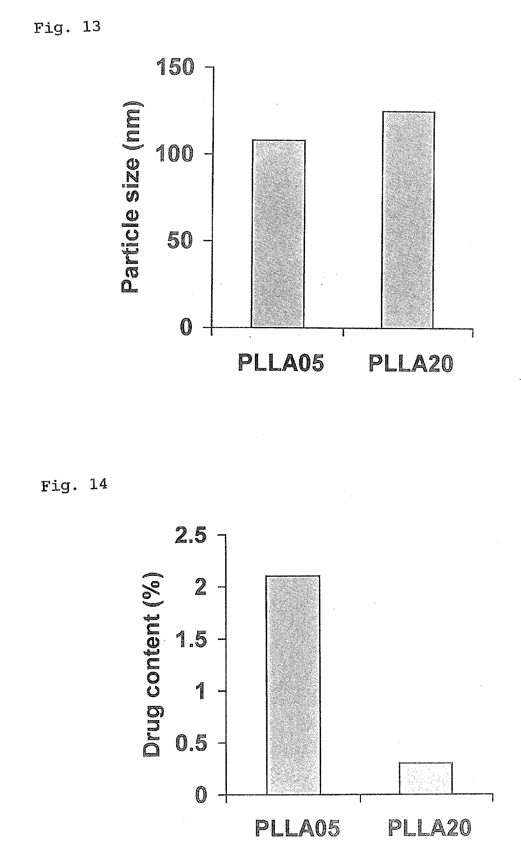

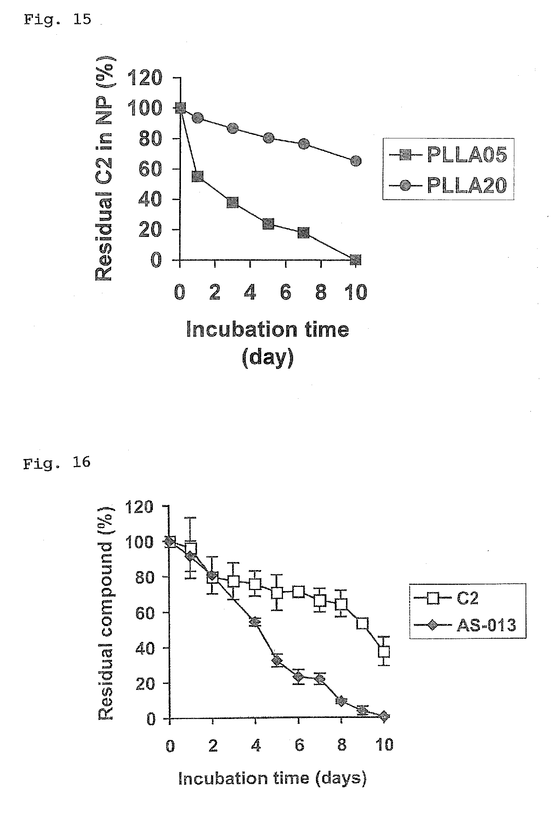

Fig. 13 is shows the particle diameter of the obtained various nanoparticles, Fig. 14 shows the encapsulation rate of the PGE1 derivative, and Fig. 15 shows the results of an in vitro release test.

The release test of PGE1 from the particles was specifically carried out based on the following method.
Nanoparticles having PGE1 encapsulated therein were dispersed in a mixed solution (50% v/v) of bovine serum albumin (FBS) and a phosphate buffer solution (PBS). The nanoparticles were incubated for respective lengths of time at 37°C, and then to 100 µL of each solution added was 900 µL of 50 mM EDTA (pH 7). The resultant mixtures were centrifuged for 30 minutes at 4°C and 50,000 x g, and then the supernatants were removed. The mixtures were charged with 1 mL of ultrapure water, and then again centrifuged. The supernatants were removed, and the amount of PGE1 in the sediments was measured by HPLC.

### Example 10: PGE1 Stability Test

A PGE1 derivative [(n = 2): PGE1(C2)PONa] aqueous solution (1 mM amount) was left (incubated) under conditions of 37°C and 4°C, respectively, and the residual amount of PGE1 derivative in the solutions over time was measured by HPLC.
In addition, as a control, the same treatments were also carried out on AS013, which is an ester derivative of PGE1.

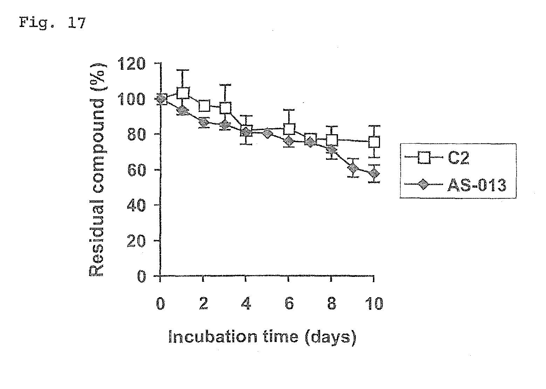

These results are shown in Figs. 16 and 17. The values shown in these tables are averages ±S.E.M. obtained by performing the test three times.
Fig. 16 shows the results of stability when incubation was carried out under 37°C conditions. Based on these results, it can be seen that the PGE1 derivative [(n = 2): PGE1(C2)PONa] according to the present invention has better stability than AS013.
Further, Fig. 17 shows the results of stability when incubation was carried out under 4°C conditions. Based on these results, it can be seen that stability is further improved under such conditions.

### INDUSTRIAL APPLICABILITY

As described above, according to the present invention, a PGE1 derivative is provided as a prodrug that has an excellent sustained PGE1 action and that is effectively converted into PGE1.
The PGE1 derivative provided by the present invention is easily converted into PGE1. Further, the conversion of the PGE1 derivative is sustained, and the PGE1 derivative has excellent storage stability. Consequently, a particular feature of the PGE1 derivative is that it exhibits a slow-release PGE1 action.

Further, the PGE1 derivative provided by the present invention can be produced as a nanoparticle. The obtained nanoparticle is very stable, and the life of the PGE1 derivative in the particle is sustained over a prolonged period. Therefore, the nanoparticle provided by the present invention has excellent drug slow-release properties. Consequently, the nanoparticle provided by the present invention is very effective and has a large industrial applicability based on its ability to achieve a sustained PGE1 action.

## Claims

1. A prostaglandin E1 derivative represented by the following formula (I): 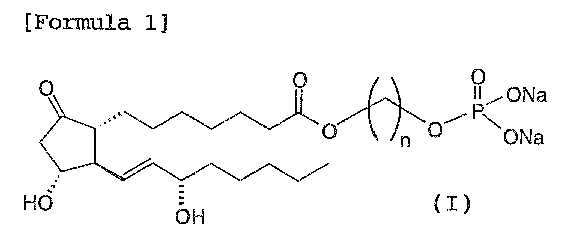 (wherein n denotes an integer of 1 to 12).

2. A prostaglandin E1 derivative represented by the formula (I), wherein n is 2.

3. A nanoparticle containing a prostaglandin E1 derivative, obtained by hydrophobicizing the prostaglandin E1 derivative represented by the formula (I) according to claim 1 with a metal ion, and reacting the hydrophobicized prostaglandin E1 derivative with poly L-lactic acid or a poly(L-lactic acid/glycolic acid) copolymer and a poly DL- or L-lactic acid-polyethylene glycol block copolymer or a poly(DL- or L-lactic acid/glycolic acid)-polyethylene glycol block copolymer.

4. The nanoparticle containing a prostaglandin E1 derivative according to claim 3, obtained by further mixing with a basic low-molecular-weight compound.

5. The nanoparticle containing a prostaglandin E1 derivative according to claim 3 or 4, obtained by further adding a surfactant.

6. The nanoparticle containing a prostaglandin E1 derivative according to claim 3, 4 or 5, wherein the metal ion is one kind or two or more kinds of a zinc ion, an iron ion, a copper ion, a nickel ion, a beryllium ion, a manganese ion, and a cobalt ion.

7. The nanoparticle containing a prostaglandin E1 derivative according to any of claims 3 to 6, wherein the poly DL-or L-lactic acid-polyethylene glycol block copolymer or the poly(DL-or L-lactic acid/glycolic acid)-polyethylene glycol block copolymer has a weight average molecular weight of 3,000 to 30,000.

8. The nanoparticle containing a prostaglandin E1 derivative according to claim 4, wherein the basic low-molecular-weight compound is one kind or two or more kinds selected from (dimethylamino)pyradine, pyridine, piperidine, pyrimidine, pyrazine, pyridazine, quinoline, quinuclidine, isoquinoline, bis(dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylamine, dimethylamine, trimethylamine, triethylenediamine, diethylenetriamine ethylenediamine, and trimethylenediamine.

9. The nanoparticle containing a prostaglandin E1 derivative according to claim 5, wherein the surfactant is one kind or two or more kinds selected from phosphatidylcholine, polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (80) octylphenyl ether, polyoxyethylene (20) cholesterol ester, lipid-polyethylene glycol, polyoxyethylene hydrogenated castor oil, and a fatty acid-polyethylene glycol copolymer.

10. The nanoparticle containing a prostaglandin E1 derivative according to any of claims 3 to 9, having a particle diameter of 20 to 300 nm.
